# EUROPEAN PATENT APPLICATION

(11) **EP 4 442 308 A1**
(43) Date of publication of application: **09.10.2024**
(21) Application number: 23179722.6
(22) Date of filing: 16.06.2023
(51) Int. Cl.: A61M 39/04, A61M 39/06

(54) **ACCESS DEVICE FOR INTRODUCING ELONGATED DEVICES INTO THE BODY OF A LIVING BEING**

(30) Priority: 03.04.2023 US 202363493856 P
(71) Applicant: Innovheart S.r.l., 20123 Milano (MI) (IT)
(72) Inventor: MCBRIDE, Mitchell, Somerville, MA, 02143 (US); FORDE, Sean, Watertown, MA, 02472 (US); BUTZIGER, John, East Greenwich, RI, 02818 (US)
(74) Representative: Provvisionato, Paolo

(57) **Abstract**

An access device for introducing elongated devices into a body of a living being comprises a hollow body having an inlet and an outlet. Valve members are provided to seal the inlet and the outlet, respectively, so as to define a confined chamber through which one or more elongated devices can be made to pass. The access device further comprises a fill port which opens in the confined chamber and through which a fluid can be made to enter in the confined chamber to fill it. The access device can be integrated into a hemostasis valve arrangement which comprises a main hemostasis valve with a main hollow body having a distal end region and a proximal end region. At least a seal element is disposed in the hollow body. The seal element defines a sealable passageway through the hollow body for the introduction of one or more operational medical devices. The outlet of the access device is in fluid communication with the sealable passageway of the main hemostasis valve.

## Description

### FIELD OF INVENTION

The present invention relates generally to systems, devices and methods for introducing elongated devices into the body of a living being.

The invention has been particularly developed to support transcatheter procedures for the therapeutic treatment of dysfunctions associated with cardiac pathologies.

The invention involves an access device which can be used to introduce elongated devices into the body of a living being, e.g. a patient. Typical elongated devices can include cannulas, catheters, cables, guidewires, prosthetic elements, tubes and other, devices which will all be named in the present description also as operational medical instruments. The operational medical instruments generally extend through the access device and have at least one distal end which is operationally located inside the body of the patient, and at least one proximal end which is operationally located outside the body of the patient.

### TECHNOLOGICAL BACKGROUND

Historically, the corrective treatment of dysfunctions related to the main cardiac pathologies has been associated with surgical procedures which are highly invasive for the patient and are frequently accompanied by high intraoperative mortality. A typical example of these highly invasive procedures is the replacement or repair of malfunctioning heart valves by means of the surgical opening of the chest, the emptying of the heart, requiring extracorporeal blood circulation, and the surgical opening of the heart itself to provide direct access to the malfunctioning heart valve.

Recently, methods of treatment and correction of cardiac pathologies have been developed with the aim of providing the same efficacy as surgical treatment, but with a drastic reduction in the invasiveness of the procedure, thereby greatly decreasing the incidence of intra- and post-operative complications and almost completely eliminating discomfort for the patient. These methods are essentially based on the use of catheters, from which the general term "transcatheter methods" is derived, as well as endoscopic instruments and special prosthetic devices. These prosthetic devices may be reduced in their overall dimensions during their introduction into the cardiac cavities via access ports with low invasiveness (for example, transfemoral, transvenous, transapical and other accesses), and then deployed in their operating configuration when the implantation site has been reached. These methods usually require a set of interventional elements, such as cables and/or guidewires and the like, which can be slidably introduced into guiding catheters and onto which other interventional elements, such as catheters and/or prosthetic elements or devices can slide and be introduced into the body of a patient to advance and complete the interventional procedure.

The introduction of elongated devices such as catheters, cables, guidewires and the like in transcatheter procedures involves making an access aperture in the body of a patient through which the catheter system is installed in order to gain access to the inner organs and cavities of a patient. It is important that the loss of bodily fluid which leaks through the access aperture is reduced to a minimum throughout the whole interventional procedure, which in the case of treatment and correction of cardiac pathologies can be long and involve repeated introductions and extractions of elongated elements of different diameter.

The catheter systems which are installed in the body of the patient make use of a valve which provides for a friction fit around the catheter to provide hemostasis, that is, preventing blood loss from a vessel or organ of the body. These kind of valves are generally known as "hemostasis valves", which are well known and used in medical procedures requiring the insertion of a catheter into the vascular system of a patient. Hemostasis valves are employed for leak-proof introduction of catheters into the circulatory system of a patient or elsewhere in the body of the patient.

In general terms, a hemostasis valve comprises a tubular, hollow body, with a distal end region and a proximal end region. Typically, a guide catheter is connected to the distal end of the hemostasis valve, and an operating instrument, such as a guidewire or balloon dilation catheter, is inserted into the proximal end and through the guide catheter to the desired location in the patient. A seal member is disposed in the tubular body of the hemostasis valve to maintain a certain seal around the operating instrument. After the operating instrument is in place, the valve closes around the operating instrument to prevent blood from escaping from the body of the patient. Hemostasis valves prevent the leakage of blood out of the ends of dilatation and guide catheters, to prevent the flow of blood between an inserted guidewire and the dilatation catheter, and also between the dilation catheter and the guide catheter.

Although the main purpose of a hemostasis valve is that of reducing blood leakage and loss, it is almost equally important that the hemostasis valve reduces the risk of air embolism, that is, the penetration of air inside the body, and particularly the blood vessels, of the patient.

As can be evident from the above, the design and manufacture of hemostasis valve are the result of a balanced compromise between the need for a tight fit around the operational instrument, on one side, in order to reduce blood loss and risk of air embolism and a relatively loose fit to allow smooth handling of the operational instrument, on the other side, to insert and retract it along the guide catheter without excessive friction.

One major disadvantage of many known hemostasis valves is that they are not able to reach a good compromise when the diameter of the operational instruments which need to pass through the sealing element of the valve is too diverse within the same interventional procedure. For example, a hemostasis valve designed for use with large catheters will inevitably result in a fit that is too loose with small diameter cables and guidewires, with the consequence that blood leaking will occur. On the contrary, a hemostasis valve optimized for small diameters of operational instruments will result in a fit that is too tight when a larger device needs to be inserted into the body of the patient.

It is to be mentioned that no known hemostasis valve is totally leak proof during catheter exchanges. In other words, there is a response time of the seal system when an instrument is introduced or removed, and during that brief time fluid can be exchanged across the hemostasis seal. For instance, when a large instrument is removed from a hemostasis valve seal, the seal closes to fill the now empty space left by the removed instrument, but it doesn't necessarily close instantaneously, leaving a brief moment of allowable fluid exchange. In fact, even the air or fluid trapped on the surface of an introduced instrument cannot be totally handled by any hemostasis valve.

Moreover, during interventional procedures there are potential situations in which the fluid pressure in the introducer column distal to the hemostasis valve seal is very low, or even zero or even negative. Particularly in these cases, air can be introduced during instrument exchange across the hemostasis valve seal, with potentially very serious consequences for the patient.

Thus, there is a general interest and need in the field of interventional procedures, particularly by means of transcatheter methods, to devise a way to address the various problems of the prior art, including the risk that air enters the body of a patient both during catheter insertion and in the persistent presence of interventional elements such as cables or guidewires traversing through the hemostasis valve between catheter exchanges.

There is also a general interest to avoid blood leaking from a hemostasis valve at various points during interventional procedures, particularly when catheters are removed or exchanged.

There is also a general interest to reduce catheter insertion friction in an easy and efficient way. There is also a general interest to reduce the cost of the materials to be used, without reducing the overall performances and quality of the access device.

It is an aim of the present invention to provide an access device for introducing elongated devices into a body of a living being, such as for example the body of a patient in a transcatheter procedure, having an improved functionality as to prevent blood leaking even when the diameter of the operational devices passing through the sealing element of the valve is greatly diverse within the same interventional procedure.

It is another aim that of providing an access device which greatly reduces or eliminates air introduction during catheter insertion as well as in the persistent presence of interventional elements, such as cables or guidewires, traversing through the access device between catheter exchanges.

It is another aim of the invention that of providing an access device which, when used in connection with a hemostasis valve, is able to reduce catheter insertion friction in an easy and efficient way.

It is a further aim of the invention that of providing an access device which, when used in connection with a hemostasis valve, is able to reduce the cost of the materials to be used for manufacturing the seal of the hemostasis valve, without reducing its overall performances and quality. It is yet another aim of the invention that of providing an access device which is simple to manufacture, set up and use in interventional operations, particularly transcatheter procedures and method, especially but not exclusively in connection with a hemostasis valve.

In order to reach these and other aims, the invention has the features set forth in the appended claims.

### BRIEF DESCRIPTION OF THE INVENTION

Aspects of the present invention include an access device which can be provided and used as a stand-alone device to access the body of a living being, e.g. a patient, by means of elongated devices passing through the access device, to reach the inside of the body from the outside thereof. Other aspects of the invention include the combination of such an access device with a hemostasis valve, where the access device functions as an antechamber to the hemostasis valve. The access device as well as methods of using it overcome the disadvantages of the prior art. These aspects of the invention can be used in a variety of diagnostic, therapeutic and interventional procedures, with a particular focus on interventional cardiology involving the use of cardiac catheter systems in transcatheter methods.

According to a first aspect, it is described an access device for introducing elongated elements into a body. In some embodiments, the access device can be provided and used as a stand-alone access device. In some other embodiments, the access device is provided and used as an antechamber to a hemostasis valve attached thereto.

The access device comprises a hollow body having an inlet and an outlet for one or more elongated devices, particularly operational medical instruments such as cannulas, catheters, cables, guidewires, prosthetic elements, tubes and other similar devices. The inlet and the outlet of the access device can be sealable by respective valve members to define a confined chamber. In case the access device is coupled to a hemostasis valve to function as an antechamber thereof, the outlet valve member of the access device can omitted because its function is taken by the seal member of the hemostasis valve, which define a seal for said confined chamber.

The confined chamber of the access device can be filled with a fluid, preferably a biocompatible and/or sterile fluid. The one or more elongated devices, particularly the operational medical instruments, can therefore pass through the confined chamber so as to be wetted therein by the fluid. The access device functions as a sort of hydraulic lock and maintains a higher hydraulic head, or "reservoir", of sterile, biocompatible fluid in order to prevent air introduction during catheter introduction.

According to an aspect, it is described an access device coupled to a hemostasis valve. The hemostasis valve can comprise a hollow body with a distal end region and a proximal end region. The hemostasis valve can comprise at least a seal element, which can be disposed in the hollow body. The seal element can define a sealable passageway through the hollow body for the introduction of one or more operational medical devices, such as catheters and the like.

When the access device is secured to a hemostasis valve, the hydraulic reservoir of fluid maintains hydraulic continuity across the hemostasis valve arrangement such that any sealing quality reduction in the hemostasis valve and subsequent loss of blood, such as due to secondary catheter introduction or due to the persistent presence of interventional elements such as cables or guidewires traversing through the access device hydraulic lock and through the hemostasis valve between catheter exchanges, will be instantaneously replenished by the sterile, biocompatible fluid contained within the confined chamber of the access device, without any air introduction into the introducer catheter. The access device hydraulic lock allows for automatic wetting of secondary catheters to reduce insertion friction as they are introduced across the hemostasis valve, without the need of manual lubrication by physician's hands with a wetted cloth.

In case the access device is coupled to a hemostasis valve, the hydraulic lock provided by the access device additionally allows for the relaxation of the seal quality requirements of the hemostasis valve due to the reduced risk of air introduction, which further reduces friction compared to a stand-alone hemostasis valve. The hydraulic lock provided by the access device may incorporate proximal seals to allow for interventional element insertion.

The access device may incorporate an atmospheric aperture to allow for pressure discharge during flushing and filling of the confined chamber thereof. The access device may further incorporate hydraulic quick-connectors to allow for easy flushing and aspiration. The access device confined chamber, acting as a fluid reservoir, may be pressurized and/or replenished via ancillary equipment commonly found in operating rooms, such as with a pressure bag or fluid-filled syringe, via standard luer fittings and/or hemostasis fittings.

According a particular aspect, the valve member or members of the access device can be of the membrane type, preferably the flat membrane type, which can be configured to open a sealable passageway at the inlet and/or outlet of the access chamber hollow body upon introduction therethrough of the elongated devices, particularly the operational medical instruments. These membrane valve or valves can have a less tight fit onto the elongated devices which are introduced through the access device, particularly when the diameter of the elongated devices is small such as for cables or guidewires. In fact, a possible leakage of some fluid from the confined chamber to the outside thereof through the inlet and/or the outlet of the access device does not compromise its functionality access device as a hydraulic lock, since the fluid remaining in the confined chamber still isolates the outside environment, upstream of the inlet of the access device, from the environment inside the body of a patient, downstream of the outlet of the access device, thus preventing leakage of body fluids, particularly blood outside the access device, or introduction of air into the body from the outside thereof.

In the event that the leakage of the biocompatible and/or sterile fluid from the confined chamber of the access device become relevant, a refilling thereof can be provided through a conduit which opens in the confined chamber and which can be connected to a source of biocompatible and/or sterile fluid to initially fill, and subsequently replenish, the confined chamber.

According to a particular aspect, the inlet flat membrane valve of the access device can be positioned at the proximal end of the hollow body thereof. This configuration allows for a compact construction of the access device, whose hollow body can thus substantially coincide with the confined chamber, to the advantage of a compact construction.

According to another particular aspect, the proximal end of the hollow body of the access device is configured with a flange, an annular member being provided at the proximal end to cooperate with said flange to block an annular edge of the flat membrane valves onto the hollow body of the access device at the inlet thereof. This provides a secure and easy way to position and block the flat membrane valve at the proximal edge of the hollow body of the access device.

According to several embodiments and other aspects, the access device mentioned above can be incorporated to a hemostasis valve so as to be integral therewith. In this case, the hemostasis valve is ready for use at the operational site, with the access device only needing to be filled with biocompatible fluid from an external source.

According to different embodiments, the access device can provided as a standalone component, which can be attached to a separate, standard hemostasis valve. In this case, greater flexibility is left to the user, who can decide whether or not to provide the hemostasis valve with the access device, according to the specific operational procedure to carry on. In these cases, the access device can be coupled to the hemostasis valve and locked thereon directly at the operational site.

Other embodiments of the hemostasis valve arrangement may include fluid level indicators or alarms, self-contained fluid replenishment, multi-ports for multiple, side-by-side interventional element insertion.

According to a particular aspect, where the access device is attached to a hemostasis valve, the seal element of the latter is disposed at the interface with the access device, right at the proximal end region of the main hemostasis valve. The seal element can be of a lesser rigidity than those of the prior art, since the access device functions as a hydraulic lock which does not allow leakage of bodily fluids from the main hemostasis valve, and prevents air form the outside to enter the main hemostasis valve.

The access device according to the present invention can be carried by any catheter or sheath introducer, to permit an inner catheter, probe, or the like to be placed through the hemostasis valve arrangement to form a leak-proof seal and a port of entry. These and additional advantages and features of the invention are clear when the attached figures are viewed in light of the accompanying descriptive matter.

### BRIEF DESCRIPTION OF THE DRAWINGS

Fig. 1 is a perspective view of an exemplary embodiment of an access device, taken from its the distal side;
Fig: 2 is a perspective view of the access device element of Fig,. 1, taken from the proximal side thereof;
Fig. 3 is an elevational view of the access device element seen from arrow III of Fig. 1;
Fig. 4 is a side view of the access device element seen from arrow IV of Fig. 3;
Fig. 5 is an elevational view of the access device element seen from the proximal side thereon, along arrow V of Fig. 1;
Fig. 6 is a cross-sectional view of the access device element, taken along line VI-VI of Fig. 5;
Fig. 7 is a schematic perspective view of a hemostasis valve assembly including the access device of Fig. 1, with an elongated member approaching thereto;
Fig: 8 is a perspective view of the hemostasis valve assembly of Fig.7, showing the elongated member partially introduced in the access device;
Fig. 9. is a perspective view of the hemostasis valve assembly of Figs. 7 and 8, showing elongated member fully introduced through the access device and entered into the hollow body of the hemostasis valve;
Fig: 10 is a perspective view of the assembly of Figs. 7 - 9, showing a second, inner elongated member, such as a catheter, emerging from the first elongated member fully introduced into the access device and entered into the hollow body of the hemostasis valve.

### DETAILED DESCRIPTION OF EXAMPLE EMBODIMENTS

In the following examples of implementations, features that enable the invention to be implemented are described. The features described may be variously combined, and are not necessarily limited to the precise form of implementation to which the drawings and description refer. In other words, a person skilled in the art who reads the following description will be able to derive information useful for knowing how to implement one or more of the described features by combining them with one or more of the other described features, without the particular wording of the description, paragraphs, sentences or drawings constituting a limitation on the possibility of isolating one or more of the described and illustrated features in order to combine them with one or more of any of the other described and illustrated features. More in detail, any combination of any two of the features expressly described herein is to be understood as expressly described, even if said features are individually extracted from the specific context in which they may be combined or combined with other, different features, taking into account the skills and knowledge of a technical expert in the field who understands the possibility of functionally combining said features without the functional contribution of the other, different features. Unless otherwise specified, each and every element, organ, means, system, component, object described and illustrated in this description is to be understood as individually described and independently modifiable, as well as separable from and/or combinable with each and every other element, organ, means, system, component, object described and illustrated. The materials, forms and functions described and illustrated are not limiting of the present invention, but are merely specified to enable a skilled person to understand and implement the invention in a preferred but not exclusive embodiment.

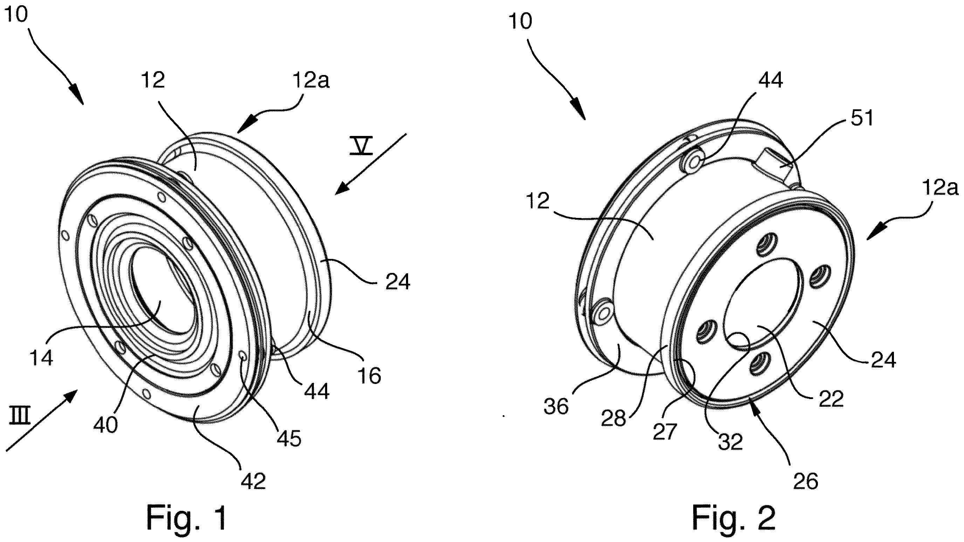

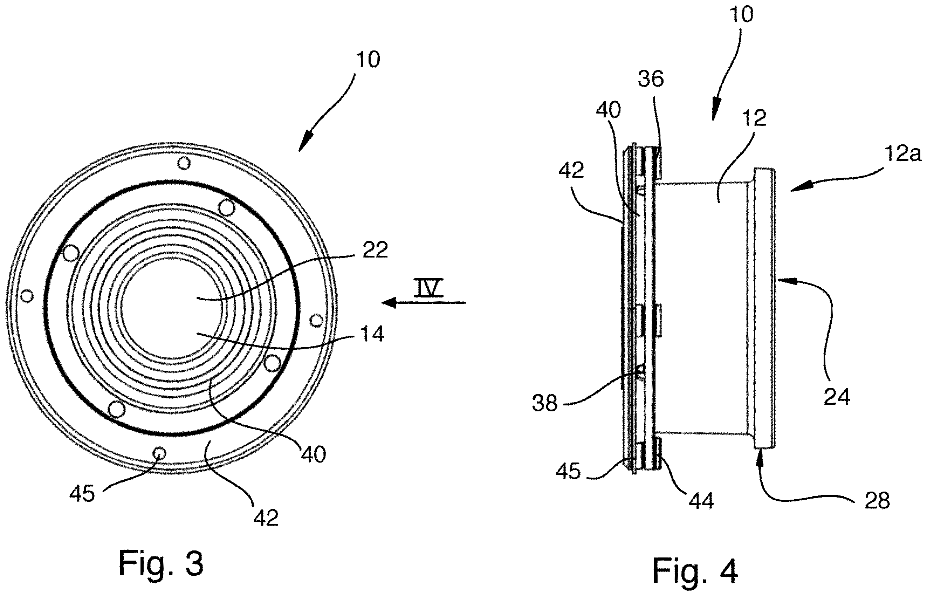

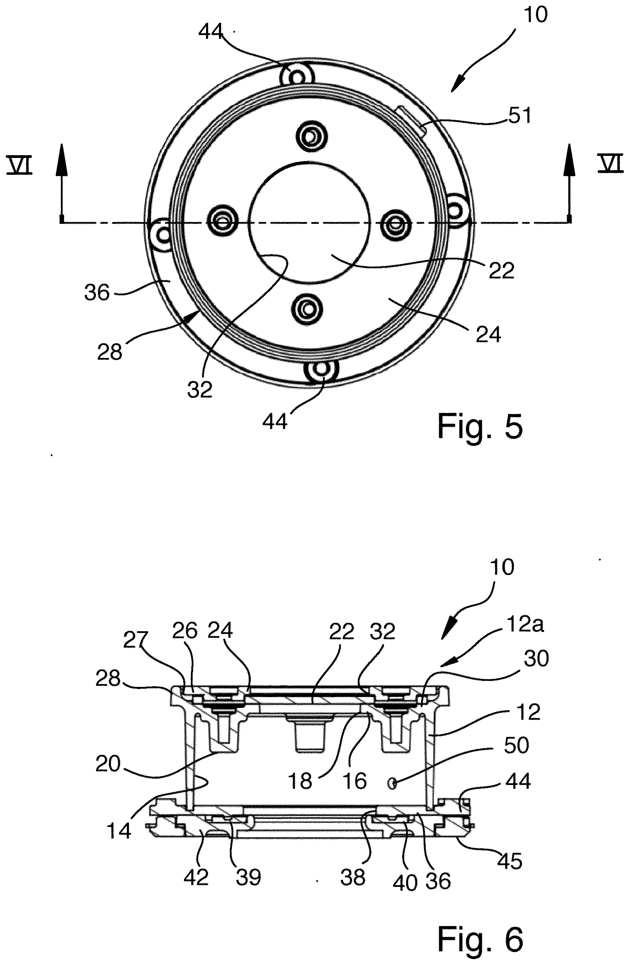

FIGS. 1 - 6 show an exemplary embodiment of an access device 10 incorporating one or more features of the present invention. The access device 10 comprises a body 12, which in the non limiting example shown in the figures has a cylindrical shape. The body 12 has an inner wall 14 which defines an inner cavity. The body 12 has a proximal edge wall 16 which partially closes one of its edges in a proximal edge area 12a. The edge wall 16 has an opening 18, preferably but non necessarily of a circular shape. A sealing membrane 22 of an elastic material, preferably but not exclusively silicone, is interposed between the proximal edge wall 16 and an outer plate 24, preferably annular in shape, so as to sealingly close the opening 18. An external edge 26 of the plate 24 is accommodated in a seat 27 made by a projecting crown 28 which extends from the edge wall 16 so as to also provide an abutment 30 for the sealing membrane 22. At regular intervals around the opening 18, for example at 90° as shown in the figures, the edge wall 16 has housings 20 for the screw threads of securing screws to assemble the plate 24 to the body 12. The plate 24 is what secures the seal membrane 22 in place. The plate 24 could also be fixed to the body 12 by adhesive bonding, ultrasonic welding, snap fit, and other known methods.

The plate 24 defines an aperture 32 which is preferably coaxial with the opening 18 of the proximal edge wall 16. The aperture 32 preferably has a wider area than the opening 18. In certain embodiments, the opening 18 of the proximal edge wall 16 and the aperture 32 of the plate 24 are circular in shape. In those embodiments, the aperture 32 has preferably a greater diameter than the opening 18.

The seal 22 is pre-perforated to allow the introduction of one or more elongate devices, particularly operational medical instruments such as catheters, cables, guidewires and the like. As an example, the pre-perforated seal 22 comprises a cross-cut. The cross-cut is done by making a linear cut more then 50% through the thickness of the seal disc on one side, and another cut on the other side offset by 90 degrees that is also cut more than 50% through the disc thickness, resulting in a central piercing when both cuts are combined and a controlled strain relief of the opening of the seal 22 on both sides.

At the distal edge of the body 12. opposed to the proximal edge wall 16, is fixed a distal plate 36. Similarly to proximal plate 24, the distal plate 36 can be screwed to the body 12 or fixed thereto by adhesive bonding, ultrasonic welding, snap fit, and other known methods. The distal plate 36 has an aperture 38, preferably of a circular shape, preferably coaxial with the opening 18 and the aperture 32. The distal plate 36 has a ridge 39, preferably annular, which is coaxial with, and surrounds the aperture 38.

The access device 10 shown in the figure is adapted to be secured to an associated hemostasis valve, as will be seen later. To this effect, the access device can be configured such that onto the ridge 39, an annular portion of a sealing ring 40 is fixed by means of an annular lid 42 which is joined to the distal plate 36. The annular lid 42 can be fixed to the distal plate 36 by screws or snap connectors 44, 45 provided at the periphery thereof, as in the examples of the figures, or any other connection means as readily understandable by the skilled person, such as by means of an adhesive bond or ultrasonic weld or snap connectors, and the like.

In alternative embodiments where the access device 10 is self-contained and is not destined to be secured to a hemostasis valve, the aperture 38 is closed by a pre-perforated membrane seal similar to the membrane seal 22 which closes the aperture 32 on proximal plate 24.

A hole 50 can be provided made through the inner wall 14 to communicate with the outside of the access device 10. The hole 50 thus opens to the atmosphere for release of air from the inner cavity during filling, so as not to create an air lock. Fluid is allowed to leak out of this hole 50 during replenishment of the access device 10. More than one hole 50 can be provided. As an alternative to the hole 50, one or more valves can be provided to release air from the inner cavity.

A refill port 51 is provided which communicates with the inner cavity of the body 12. In use, the refill port 51 is connected to a source of fluid, e.g. a biocompatible, sterile fluid to fill or replenish the inner cavity of the body 12, i.e. the confined chamber of the access device 10. As a suitable fluid, saline can for example be used.

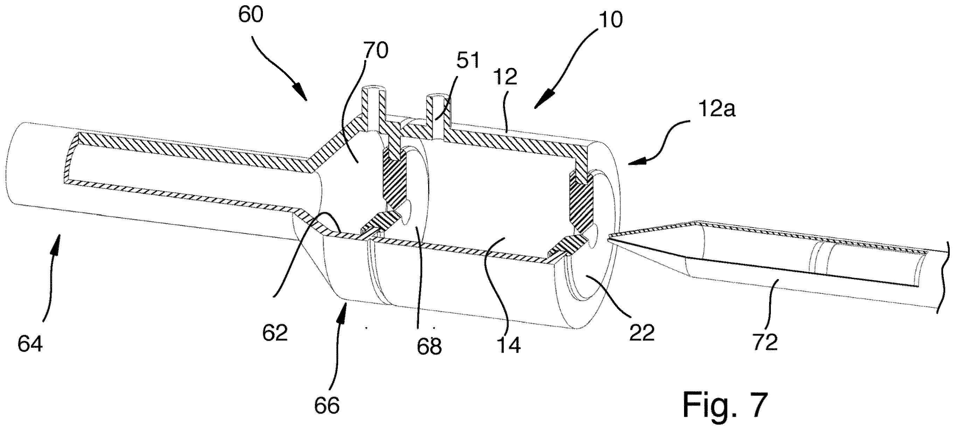

As can be seen in the schematic Figures 7-10, the access device 10 described above is secured to a hemostasis valve 60. The hemostasis valve 60 can have any configuration of a known type, which generally includes a hollow body 62 with a distal end region 64 and a proximal end region 66. At least a seal element 68 is disposed in the hollow body 62 to define a sealable passageway 70 through the hollow body 62 for the introduction of one or more operational medical instruments, such as an introducer catheter 72 as shown in the figures.

In the embodiment shown in Figures 7-10, the seal element 68 is placed at the interface between the access device 10 and the hemostasis valve 60, and is the only sealing element of the latter. In other embodiments, one or more further sealing elements can be disposed in the hollow body 62 between the proximal end region 68 and the distal end region 64, according to known designs of hemostasis valves. However, the presence of the access device 10 allows for a simplified design of the hemostasis valve 60, thanks to the additional and improved sealing properties provided by the fluid inside the inner chamber of the access device 10, as explained before.

In use, as shown in Figure 7, the access device 10 is first filled with a fluid, preferably a biocompatible, sterile fluid, such as a saline solution commonly in use in interventional procedures. In this respect, the access device 10 may be pressurized and/or replenished via ancillary equipment commonly found in operating rooms, such as with a pressure bag or fluid-filled syringe, via standard luer and hemostasis fittings, or the like. The inner chamber of the access device 10 is closed by the sealing membrane 22 at its proximal end. When it is secured to a hemostasis valve 60, the distal end of the access device 10 is also closed by the proximal membrane valve 68 of the hemostasis valve, so that the biocompatible fluid can remain confined during use in the inner cavity of the access device 10.

As shown in figure 7, an elongated device 72, for example an introducer catheter or any other desired operational medical instruments, can be introduced into the inner cavity of the access device 10 by pushing its edge through the sealing membrane 22, which opens under the pressure exercised by the tip of the elongated device 72. At this stage, some of the fluid contained in the inner cavity of the access device 10 can leak out of the access device 10 through the sealing membrane 22, although in a very small quantity. Any such lost fluid in the inner cavity of the access device 10 is quickly replenished by the refill port 51 that is attached to a sterile biocompatible fluid reservoir, such as a pressure bag. In terms of leakage, it should be mentioned that the pressurized head allows for fluid introduction from the access device 10 across the hemostasis valve 60, so that blood does not leak into the access device 10. On the contrary, it is the fluid contained in the inner cavity of the access device 10 which enters across the hemostasis valve 60. In fact, a higher pressure head of the replenishable fluid reservoir in the access device 10 maintains pressure across the hemostasis seal 68. In this way, blood is kept out of the access device 10, while air is prevented from being introduced across the hemostasis valve 60. This brings the additional advantage of relaxing the hemostasis sealing requirements.

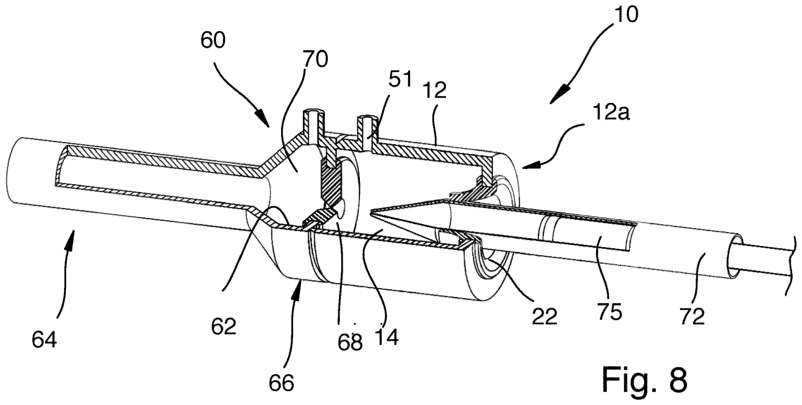

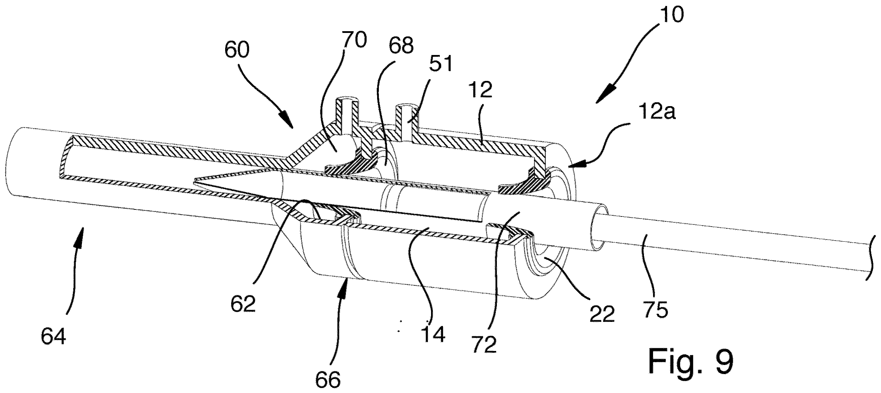

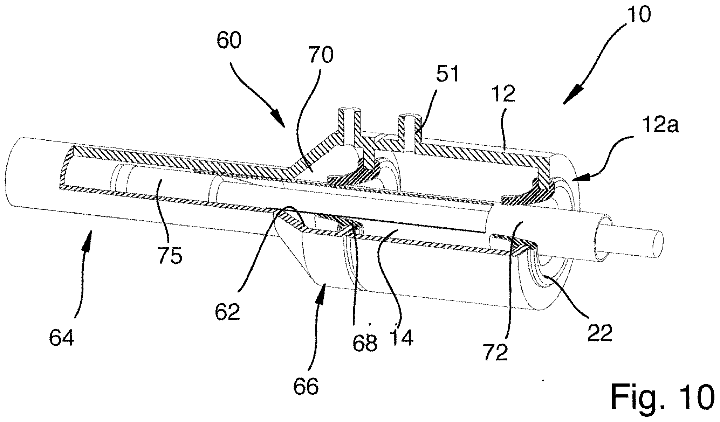

As shown in Figure 8, the elongated device 72 can be pushed further through the inner cavity of the access device 10, until it reaches the sealing membrane 68 at the interface between the access device 10 and the hemostasis valve 60. The fluid contained in the inner cavity of the access device 10 automatically wets the outer surface of the elongated device 72, so that its subsequent introduction into the hemostasis valve 60, as shown in figure 9, is facilitated. When the elongated device 72 is through the hemostasis valve 60, it can be used as a guide for a secondary elongated element 75 such as a secondary catheter or a smaller cable or guidewire, as shown in figure 10.

If the elongated device 72 is completely extracted from the hemostasis valve 60 and the access device 10, for example during the to temporary exchange of catheters, thus leaving only smaller interventional elements such as cables or guidewires traversing through the access device 10 and through the hemostasis valve 60, any possible loss of blood will be immediately replenished by the sterile, biocompatible fluid contained within the access device 10, without any air introduction.

It should be understood that with the fluid filling of the inner cavity of the access device 10, a hydraulic head, that is, a pressure higher than the fluid that is distal to the hemostasis valve seal, builds up within the access device 10. This higher pressure fluid is the hydraulic driver that pushes fluid across the valve, rather than allowing potentially higher pressure atmospheric air pass the hemostasis valve.

The access device 10 also allows for automatic wetting of elongated devices, such as for example secondary catheters, to reduce insertion friction as they are introduced across the hemostasis valve 60, without the need of manual lubrication by physician's hands with a wetted cloth.

Other possible embodiments may include fluid level indicators or alarms, self-contained fluid replenishment, multi-ports for multiple, side-by-side interventional element insertion.

The embodiments and details of the invention may vary widely from what is described and illustrated above, which is given as a mere enabling example, without departing from the scope of the present invention.

## Claims

1. An access device for introducing elongated devices into a body of a living being, the access device comprising a hollow body having an inlet and an outlet, valve members being provided to seal the inlet and the outlet, respectively, so as to define a confined chamber through which one or more elongated devices can be made to pass, the access device further comprising a fill port which opens in the confined chamber and through which a fluid can be made to enter in the confined chamber to fill it.

2. An access device as claimed in Claim 1, including an attachment arrangement for securing to a proximal end region of a hemostasis valve in a hemostasis valve arrangement.

3. An access device as claimed in Claim 1 or Claim 2, wherein the valve members are flat membrane valves, configured to open a sealable passageway at the inlet and the outlet of the access device hollow body upon introduction therethrough of said elongated devices.

4. An access device as claimed in Claim 3, wherein the flat membrane valve is positioned at the proximal end of the hollow body of the access device.

5. An access device as claimed in Claim 4, wherein the proximal end of the hollow body of the access device is configured with a flange, an annular member being provided at the proximal end to cooperate with said flange to block an annular edge of the flat membrane valve onto the hollow body of the access device at the inlet thereof.

6. An access device as claimed in any one of Claims 1 - 5, wherein the confined chamber includes a hole which opens to the atmosphere to allow for air venting from the confined chamber during the filling thereof with the fluid.

7. An access device as claimed in in any one of Claims 1 - 6, including a source of a biocompatible fluid connected through a conduit to the fill port.

8. A hemostasis valve arrangement, comprising:
- a main hemostasis valve comprising a main hollow body with a distal end region and a proximal end region, at least a seal element disposed in the hollow body, the seal element defining a sealable passageway through the hollow body for the introduction of one or more operational medical devices,
- an access device for introducing elongated devices into a body of a living being, the access device comprising a hollow body having an inlet and an outlet, the outlet being in fluid communication with the sealable passageway of the main hemostasis valve, at least a valve member being provided to seal at least the inlet, so as to define with at least the seal element of the main hemostasis a confined chamber through which one or more elongated devices can be made to pass, the access device further comprising a fill port which opens in the confined chamber and through which a fluid can be made to enter in the confined chamber to fill it.

9. A hemostasis valve arrangement as claimed in Claim 8, wherein an attachment arrangement is provided on the access device for securing the access device to the proximal end region of the main hemostasis valve.

10. A hemostasis valve arrangement as claimed in Claim 8 or Claim 9, wherein the at least one valve member of the access device is a flat membrane valve, configured to open a sealable passageway at the inlet of the access device hollow body upon introduction therethrough of said elongated devices.

11. A hemostasis valve arrangement as claimed in Claim 10, wherein the flat membrane valve is positioned at the proximal end of the hollow body of the access device.

12. A hemostasis valve arrangement as claimed in Claim 11, wherein the proximal end of the hollow body of the access device is configured with a flange, an annular member being provided at the proximal end to cooperate with said flange to block an annular edge of the flat membrane valve onto the hollow body of the access device at the inlet thereof.

13. A hemostasis valve arrangement as claimed in any one of Claims 8 - 12, wherein the confined chamber includes a hole which opens to the atmosphere to allow for air venting from the confined chamber during the filling thereof with the fluid.

14. A hemostasis valve arrangement as claimed in any one of Claims 8 - 13, including a source of a biocompatible fluid connected through a conduit to the fill port of the access device.

15. A method for using a hemostasis valve arrangement, the method comprising:
- securing a hemostasis valve arrangement to a medical device, the hemostasis valve arrangement comprising:
- a main hemostasis valve comprising a main hollow body with a distal end region and a proximal end region, at least a seal element disposed in the hollow body, the seal element defining a sealable passageway through the hollow body for the introduction of one or more operational medical devices,
- an access device for introducing elongated devices into a body of a living being, the access device comprising a hollow body having an inlet and an outlet, the outlet being in fluid communication with the sealable passageway of the main hemostasis valve, at least a valve member being provided to seal at least the inlet, so as to define with at least the seal element of the main hemostasis a confined chamber through which one or more elongated devices can be made to pass, the access device further comprising a fill port which opens in the confined chamber and through which a fluid can be made to enter in the confined chamber to fill it;
- filling the confined chamber with a biocompatible fluid; and
- introducing one or more operational medical devices in the confined chamber through the valve member at the inlet of the confined chamber, the confined chamber, the seal element of the main hemostasis valve and the sealable passageway thereof.

16. The method of claim 15, comprising the additional step of refilling the confined chamber with a biocompatible fluid through a conduit provided in the access device which opens in the confined chamber.
